**Europäisches Patentamt**

**European Patent Office**

**Office européen des brevets**

(11) Veröffentlichungsnummer : **0 360 981 B1**

# EUROPÄISCHE PATENTSCHRIFT

(45) Veröffentlichungstag der Patentschrift :
**20.05.92 Patentblatt 92/21**

(51) Int. Cl.$^5$ : **B01J 4/00,** B01J 8/02,
// C07C29/15

(21) Anmeldenummer : **89110439.0**

(22) Anmeldetag : **09.06.89**

(54) **Reaktor zur Durchführung katalytischer Gasreaktionen mit einem druckfesten Mantel und je einem Kugelboden am stirnseitigenAussenrand.**

(30) Priorität : **29.08.88 DE 3829215**

(43) Veröffentlichungstag der Anmeldung :
**04.04.90 Patentblatt 90/14**

(45) Bekanntmachung des Hinweises auf die
Patenterteilung :
**20.05.92 Patentblatt 92/21**

(84) Benannte Vertragsstaaten :
**BE CH DE ES FR GB IT LI NL**

(56) Entgegenhaltungen :
**GB-A- 2 065 492
US-A- 2 961 304
US-A- 3 915 847**

(73) Patentinhaber : **Uhde GmbH
Friedrich-Uhde-Strasse 15
W-4600 Dortmund 1 (DE)**

(72) Erfinder : **Bähnisch, Hans-Joachim
Wembersweg 30
W-4600 Dortmund 76 (DE)**

(74) Vertreter : **Patentanwälte Meinke und
Dabringhaus Dipl.-Ing. J. Meinke Dipl.-Ing. W.
Dabringhaus
Westenhellweg 67
W-4600 Dortmund 1 (DE)**

## Beschreibung

Die Erfindung betrifft einen Reaktor zur Durchführung katalytischer Gasreaktionen mit einem druckfesten Mantel und je einem Kugelboden am stirnseitigen Außenrand, wobei wenigstens im Bereich des Mantels ein Katalysatorbett angeordnet ist, über dessen Höhe eine Mehrzahl von in das Katalysatorbett mündenden Kaltgaszuführungen vorgesehen ist.

Aus der DE-A-1 542 209 ist ein derartiger Reaktor bekannt, bei dem über der Höhe des zylindrischen Mantels angeordnete Kaltgaszuführungen aus quer zur Strömungsrichtung des Reaktionsgases angeordneten, Mischraüme bildenden Käfigen mit perforierten Wänden bestehen, die vorzugsweise in ihrem Inneren angeordnete Rohre für die Zuleitung des Kaltgases aufweisen, wobei zwecks Ermöglichung des Eintritts eines wesentlichen Anteils des Reaktionsgases in die Käfige diese entsprechend groß gestaltet sind. Durch die Größe dieser Käfige wird ein relativ großer Raumbedarf für die Kaltgaszuführungseinbauten benötigt, so daß der für den Katalysator zur Verfügung stehende Raum und somit der Reaktionsraum unnötig begrenzt ist. So kann zwar eine relativ befriedigende Temperaturführung im Reaktor erreicht werden, die Leistung des Reaktors ist aber durch den begrenzt zur Verfügung stehenden Reaktionsraum nicht zufriedenstellend.

Demgegenüber besteht die Aufgabe der Erfindung in der Schaffung einer Lösung, mit welcher der mit Katalysator gefüllte Reaktionsraum eines derartigen Reaktors vergrößert werden kann, wobei auch im Zusatzraum eine möglichst gleichmäßige Temperaturverteilung einstellbar sein soll.

Diese Aufgabe wird mit einem Reaktor der eingangs bezeichneten Art erfindungsgemäß dadurch gelöst, daß im Bereich der Kugelböden je ein Katalysatorbett mit Kaltgaszuführungen angeornet ist, wobei die Kaltgaszuführungen als senkrecht angeordnete Düsenrohre mit im wesentlichen waagerechten Zuleitungen ausgebildet sind.

Durch die zusätzliche Anordnung von Katalysatorbetten im Bereich der Kugelböden wird eine wesentliche Vergrößerung der Reaktionsraumes ermöglicht, wobei durch die Ausbildung von Kaltgaszuführungen auch im Bereich der Kugelböden eine sehr gleichmäßige Temperaturverteilung auch in diesem Bereich möglich ist. Der Einsatz von Kaltgaszuführungen im Bereich der Kugelböden, welche nur einen begrenzten Innenraum aufweisen, ist nur durch die erfindungsgemäße, sehr raumsparende Ausgestaltung möglich. Bekannte Kaltgaszuführungen wie in der DE-A-1 542 209 sind aufgrund ihres großen Raumbedarfes nicht in übliche Kugelböden einbringbar. Durch die Ausbildung von Düsenrohren wird eine sehr feine Kaltgasverteilung erzielt, wodurch sich eine besonders gute Vermischung mit den heißen Reaktionsgasen und dadurch eine gleichmäßige Temperaturverteilung einstellt.

Es ist besonders zweckmäßig, wenn die Düsenrohre im oberen Kugelboden nach oben gerichtet und die Düsenrohre im unteren Kugelboden nach unten gerichtet an den jeweiligen Zuleitungen angeordnet sind. Durch diese Anordnung kann der im jeweiligen Kugelboden zur Verfügung stehende Raum optimal ausgenutzt werden, d.h. trotz des nur gering zur Verfügung stehenden Innenraumes im Bereich der Kugelböden kann eine ausreichende Menge an Kaltgas fein verteilt an das jeweilige Katalysatorbett und somit an das heiße Reaktionsgas abgegeben werden.

Konstruktiv besonders einfach und raumsparend sieht die Erfindung vor, daß die Zuleitungen als zu beiden Seiten eines Zentralrohres mit mittig angeordnetem, den Kugelbodenmantel durchdringendem Kaltgaszuführungsrohr angeordnete Verteilerrohre ausgebildet sind. Dabei werden die Kaltgaszuführungen vorzugsweise so installiert, daß die Verteilerrohre etwa im Übergangsbereich zwischen dem zylindrischen Reaktormantel und dem jeweiligen Kugelboden angeordnet sind. Diese kompakte Ausgestaltung eignet sich insbesondere auch zur Nachrüstung von Reaktoren.

Besonders vorteilhaft ist vorgesehen, daß die außenrandseitigen Düsenrohre kürzer als die inneren Düsenrohre ausgebildet sind. Durch diese an die Halbkugelwandfläche des jeweiligen Kugelbodens angepaßte Ausbildung kann der Kugelbodenraum noch besser ausgenutzt werden.

Es ist zweckmäßig, wenn die Verteilerrohre in einem einen Katalysatordurchfluß ermöglichenden Abstand nebeneinander am Zentralrohr angeordnet sind. Der Katalysator kann dann beim Befüllen bzw. Entleeren frei durch den Zwischenraum zwischen den Verteilerrohren hindurchfließen.

Schließlich sieht die Erfindung auch noch vor, daß die Düsenrohre von einer perforierten Ummantelung umgeben sind, welche bevorzugt aus Streckmetall gebildet ist. Durch diese Ummantelung wird einerseits ein Verstopfen der Düsenöffnungen der Düsenrohre durch Katalysator vermieden, andererseits ist gewährleistet, daß das ausströmende Kaltgas unbehindert in das Katalysatorbett eintreten kann.

Die Erfindung ist nachstehend anhand der Zeichnung beispielsweise näher erläutert. Diese zeigt in:

Fig. 1 in stark vereinfachter Darstellung einen Schnitt durch einen erfindungsgemäß ausgebildeten Reaktor,

Fig. 2 einen Querschnitt durch den Reaktor im Bereich der Kugelböden,

Fig. 3 ein vergrößert dargestelltes Düsenrohr in einer Seitenansicht und

Fig. 4 einen Schnitt gemäß der Linie IV - IV in Fig. 3.

Ein allgemein mit 1 bezeichneter Reaktor ist insbesondere zur Methanolsynthese bei hohen Drücken und Temperaturen zwischen 200 und 300° C geeignet, selbstverständlich kann dieser Reaktor aber auch für andere exotherme oder auch endotherme katalytische Gasreaktionen eingesetzt werden.

Der Reaktor 1 ist in der Zeichnung nur mit den für die Erfindung wesentlichen Merkmalen dargestellt. Der Reaktor weist einen zylindrischen Mantel 2, einen oberen Kugelboden 3 mit Gaseinlaßstutzen 4 und einen unteren Kugelboden 5 mit Gasauslaßstutzen 6 auf. Im Bereich des zylindrischen Mantels 2 ist ein Katalysatorbett 7 angeordnet, wobei über der Höhe des Katalysatorbettes 7 eine Mehrzahl von in das Katalysatorbett mündenden Kaltgaszuführungen vorgesehen ist, die in der Zeichnung nicht näher dargestellt sind und auf die es hier auch nicht näher ankommt.

Im Bereich des oberen Kugelbodens 3 sowie im Bereich des unteren Kugelbodens 5 ist jeweils ein zusätzliches Katalysatorbett 7a, 7b mit Kaltgaszuführungen 8a, 8b vorgesehen. Dabei sind die zusätzlichen Katalysatorbetten 7a, 7b in der Zeichnung als Einzelbetten dargestellt, selbstvertändlich können die Katalysatorbetten aber auch direkt mit dem Katalysatorbett 7 verbunden sein, d.h. als gemeinsames, den gesamten Reaktor 1 im wesentlichen ausfüllendes Katalysatorbett ausgebildet sein.

Die Kaltgaszuführungen 8a, 8b sind von senkrecht angeordneten Düsenrohren 9a, 9b mit im wesentlichen waagerechten Zuleitungen 10a, 10b gebildet. Die Düsenrohre 9a im oberen Kugelboden 3 sind dabei senkrecht nach oben gerichtet und die Düsenrohre 9b im unteren Kugelboden 5 senkrecht nach unten gerichtet, wobei bevorzugt jeweils die außenrandseitigen Düsenrohre kürzer ausgebildet sind, d.h. an die gekrümmte Wandfläche der Kugelböden angepaßt sind, so daß eine optimale Raumausnutzung möglich ist.

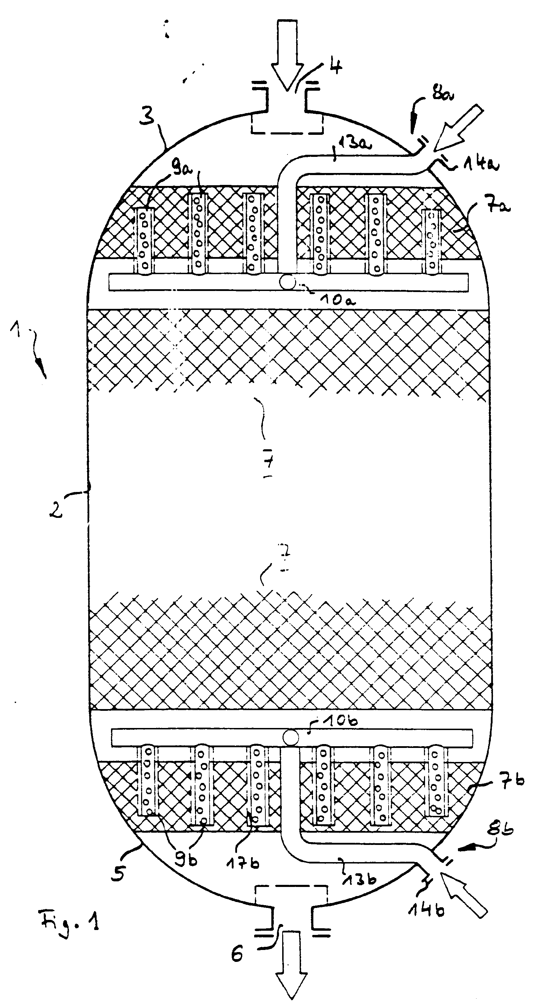

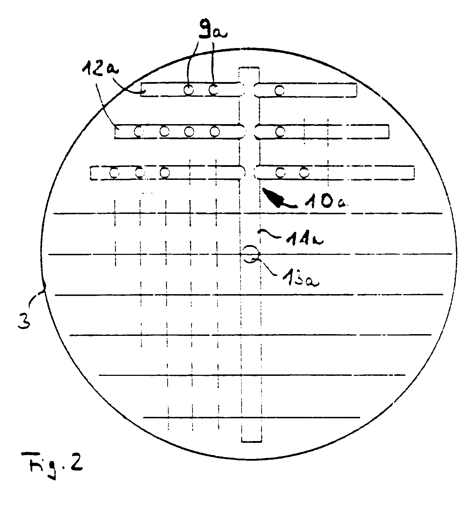

Wie besonders gut aus Fig. 2 hervorgeht, bestehen die Zuleitungen 10a aus einem Zentralrohr 11a mit zu beiden Seiten desselben nebeneinander angeordneten Verteilerrohren 12a, an denen die Düsenrohre 9a angeordnet sind. Das Zentralrohr 11a ist mittig mit einem Kaltgaszuführungsrohr 13a verbunden, welches den Mantel des oberen Kugelbodens 3 durchdringt und am freien Ende einen Kaltgasanschlußstutzen 14a aufweist. Die Zuleitungen 10b im Bereich des unteren Kugelbodens 5 sind entsprechend ausgebildet, wobei in der Fig. 1 dieselben Bezugszeichen mit dem Zusatz "b" verwandt sind.

Wie aus Fig. 2 hervorgeht, sind die einzelnen Verteilerrohre derart nebeneinander angeordnet, daß der lichte Abstand zwischen den einzelnen Verteilerrohren ausreichend groß ist, um einen Katalysatordurchfluß beim Befüllen oder Entleeren zu ermöglichen. Es hat sich herausgestellt, daß ein lichter Abstand von etwa 80 mm besonders günstig ist, wobei dieser Abstand selbstverständlich auch von der Korngröße des Katalysators abhängt.

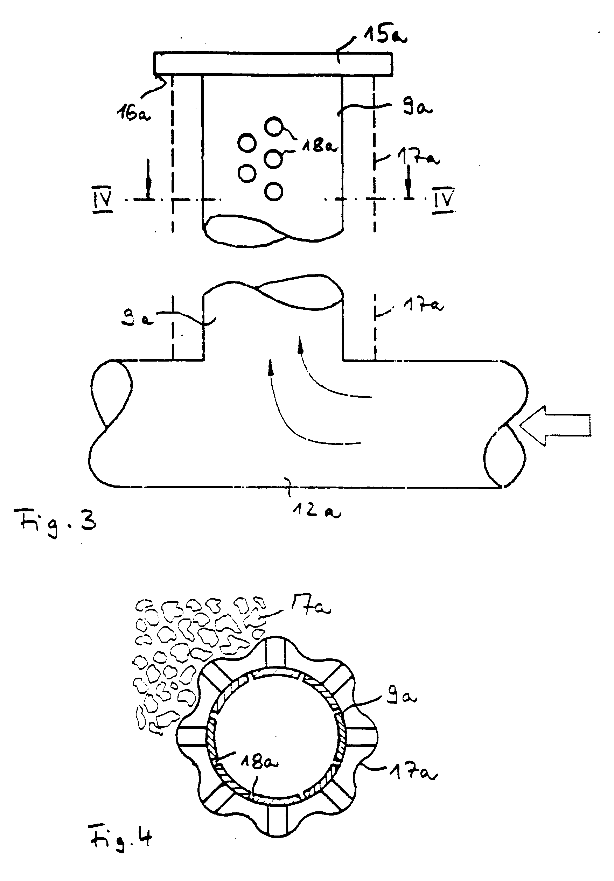

Aus den Fig. 3 und 4 ist der Aufbau eines Düsenrohres zu erkennen, wobei nur ein Düsenrohr 9a dargestellt ist, die Düsenrohre 9b im unteren Kugelbodenbereich sind entsprechend ausgestaltet. Jedes Düsenrohr 9a weist am freien Ende eine scheibenförmige Abdeckung 15a auf, deren Außendurchmesser größer als der Außendurchmesser des Düsenrohres 9a ausgebildet ist, so daß eine Auflagefläche 16a für eine perforierte Ummantelung 17a ausgebildet ist. Diese Ummantelung ist bevorzugt aus Streckmetall gebildet. und umhüllt den Außenbereich des Düsenrohres 9a, in welchem eine Vielzahl von Düsenöffnungen 18a ausgebildet sind. Durch die Ummantelung wird vermieden, daß sich die Düsenöffnungen 18a mit Katalysator zusetzen und verstopen können.

Wie auf Fig. 1 hervorgeht, sind die Kaltgaszuführungen 8a, 8b derart im Reaktor 1 angeordnet, daß die Zuleitungen 10a, 10b sich im Übergangsbereich vom zylindrischen Mantel 2 zu den Kugelböden 3, 5 befinden. Bei der Befüllung des Reaktors 1 mit Katalysator wird zunächst der zylindrische Bereich 2 mit dem unteren Kugleboden 5 befüllt und anschließend der Bereich des oberen Kugelbodens 3, wobei zum Ausgleich von späteren Katalysatorschrumpfungen der obere Kugelboden 3 zunächst höher befüllt wird, als in der Zeichnung dargestellt ist.

Die Reaktionsgase werden durch den Gaseinlaßstutzen 4 in den Reaktor 1 eingeleitet und strömen von oben nach unten im wesentlichen senkrecht, zunächst durch das obere Katalysatorbett 7a, das Katalysatorbett 7 und schließlich durch das untere Katalysatorbett 7b, um den Reaktor durch den Gasauslaßstutzen 6 zu verlassen. Eine Temperaturerhöhung im Katalysatorbett 7a, 7, 7b beim Ablauf der Reaktion wird durch die Einleitung von Kaltgas vermieden, wobei im zylindrischen Bereich 7 in bekannter weise Kaltgaszuführungen vorgesehen sind, selbstverständlich können auch den Kaltagaszuführungen 8a, 8b entsprechende Kaltgaszuführungen verwandt werden. Im oberen Kugelboden 3 wird das Kaltgas durch den Kaltagasanschlußstutzen 14a eingeleitet und strömt durch das Kaltgaszuführungsrohr 13a in das Zentralrohr 11a und von dort über die einzelnen Verteilerrohre 12a in die Düsenrohre 9a. Durch die Düsenöffnungen 18a tritt das Kaltgas fein verteilt senkrecht zur Hauptströmungsrichtung des heißen Reaktionsgases in das Katalysatorbett 7a ein und vermischt sich mit dem Reaktionsgas, so daß sich eine gleichmäßige Temperaturverteilung im Katalysatorbett 7a einstellt. Ein gleichmäßiges Austreten des Kaltgases durch die Düsenöffnungen 18a wird dadurch gewährleistet, daß das Kaltgas mit einem entsprechend hohen Druck in das Kaltgaszuführungsrohr 13a eingeleitet wird. Die Kaltgaszuführung im unteren Kugelboden 5 erfolgt analog.

Natürlich ist die Erfindung nicht auf die in der Zeichnung dargestellten Ausführungsbeispiele beschränkt. Weitere Ausgestaltungen der Erfindung sind möglich, ohne den Grundgedanken zu verlassen. So kann in bestimmten Einsatzfällen auch nur im oberen Kugelboden 3 ein zusätzliches Katalysatorbett 7a vorgesehen sein, wie auch nur im unteren Kugelboden 5 und dergl. mehr.


**Patentansprüche**

1. Reaktor zur Durchführung katalytischer Gasreaktionen mit einem druckfesten Mantel und je einem Kugelboden am stirnseitigen Außenrand, wobei wenigstens im Bereich des Mantels ein Katalysatorbett angeordnet ist, über dessen Höhe eine Mehrzahl von in das Katalysatorbett mündenden Kaltgaszuführungen vorgesehen ist,
dadurch gekennzeichnet,
daß im Bereich der Kugelböden (3,5) je ein Katalysatorbett (7a,7b) mit Kaltgaszuführungen (8a,8b) angeordnet ist, wobei die Kaltagaszuführungen (8a,8b) als senkrecht angeordnete Düsenrohre (9a,9b) mit im wesentlichen waagerechten Zuleitungen (10a,10b) ausgebildet sind.

2. Reaktor nach Anspruch 1,
dadurch gekennzeichnet,
daß die Düsenrohre (9a) im oberen Kugelboden (3) nach oben gerichtet und die Düsenrohre (9b) im unteren Kugelboden (5) nach unten gerichtet an den jeweiligen Zuleitungen (10a,10b) angeordnet sind.

3. Reaktor nach Anspruch 1 oder 2,
dadurch gekennzeichnet,
daß die Zuleitungen (10a,10b) als zu beiden Seiten eines Zentralrohres (11a,11b) mit an diesem mittig angeordnetem, den Kugelbodenmantel (3,5) durchdringendem Kaltgaszuführungsrohr (13a,13b) angeordnete Verteilerrohre (12a,12b) ausgebildet sind.

4. Reaktor nach Anspurch 1 oder einem der folgenden,
dadurch gekennzeichnet,
daß die außenrandseitigen Düsenrohre (9a,9b) kürzer als die inneren Düsenrohre (9a,9b) ausgebildet sind.

5. Reaktor nach Anspruch 1 oder einem der folgenden,
dadurch gekennzeichnet,
daß die Verteilerrohre (12a,12b) in einem einen Katalysatordurchfluß ermöglichenden Abstand nebeneinander am Zentralrohr (11a,11b) angeordnet sind.

6. Reaktor nach Anspruch 1 oder einem der folgenden,
dadurch gekennzeichnet,
daß die Düsenrohre (9a,9b) von einer perforierten Ummantelung (17a) umgeben sind.

7. Reaktor nach Anspruch 6,
dadurch gekennzeichnet,
daß die Ummantelung (17a,17b) aus Streckmetall gebildet ist.


**Claims**

1. Reactor for carrying out catalytic gas reactions with a pressure-resistant shell and a spherical base at each end, wherein a catalyst bed is located at least in the region of the shell, over whose height a plurality of cold gas feeds opening into the catalyst bed are distributed,
characterised in that
a catalyst bed (7a, 7b) with cold gas feeds (8a, 8b) is located in the region of each of the spherical bases (3, 5), the cold gas feeds (8a, 8b) being formed as vertically mounted nozzle tubes (9a, 9b) with substantially horizontal supply lines (10a, 10b)

2. Reactor according to claim 1,
characterised in that
the nozzle tubes (9a) in the upper spherical base (3) are oriented upwards and the nozzle tubes (9b) in the lower spherical base (5) are oriented downwards on the respective supply lines (10a, 10b).

3. Reactor according to claim 1 or 2,
characterised in that
the supply lines(10a, 10b) are formed as manifolds (12a, 12b) mounted on both sides of a central pipe (11a, 11b) with a cold gas feed pipe (13a, 13b) mounted centrally thereon and penetrating the spherical base shell (3, 5).

4. Reactor according to claim 1 or one of the following,
characterised in that
the nozzle tubes (9a, 9b) near the outer edge are shorter than the inner nozzle tubes (9a, 9b).

5. Reactor according to claim 1 or one of the following,
characterised in that
the manifolds ( 12a, 12b) are located adjacent to one another on the central pipe (11a, 11b) at a distance permitting the catalyst to flow through.

6. Reactor according to claim 1 or one of the following,
characterised in that
the nozzle tubes (9a, 9b) are surrounded by a perforated casing (17a).

7. Reactor according to claim 6,
characterised in that
the casing (17a, 17b) is formed of expanded metal.


## Revendications

1. Réacteur pour l'exécution de réactions catalytiques en phase gazeuse comprenant une chemise résistante à la pression et une partie de fond sphérique à chaque extrémité frontale, un lit de catalyseur étant disposé au moins dans la région de la chemise, au-dessus de la hauteur duquel est prévue une multiplicité de conduites d'amenée de gaz froid débouchant dans le lit de catalyseur,
caractérisé en ce que
l'on dispose dans la région de chacune des parties de fond sphériques (3, 5) un lit de catalyseur respectif (7a, 7b) avec des conduites d'amenée de gaz froid (8a, 8b), ces dernières étant constituées sous forme de tubes gicleurs (9a, 9b) disposés verticalement et comprenant des conduites (10a, 10b) sensiblement horizontales.

2. Réacteur selon la revendication 1,
caractérisé en ce que
les tubes gicleurs (9a) de la partie de fond sphérique supérieure (3) sont orientés vers le haut et les tubes gicleurs (9b) de la partie de fond sphérique inférieure (5) sont dirigés vers le bas, en partant des conduites respectives (10a, 10b).

3. Réacteur selon la revendication 1 ou 2,
caractérisé en ce que
les conduites (10a, 10b) sont constituées sous forme de tubes distributeurs de chaque côté d'un tube central sur lequel est raccordé en son centre un tube d'amenée de gaz froid qui traverse la chemise (3, 5) de la partie de fond sphérique.

4. Réacteur selon la revendication 1 ou l'une des suivantes,
caractérisé en ce que
les tubes gicleurs (9a, 9b) situés sur le côté extérieur sont plus courts que les tubes gicleurs internes (9a, 9b).

5. Réacteur selon la revendication 1 ou l'une des suivantes,
caractérisé en ce que
les tubes distributeurs (12a, 12b) sont disposés sur le tube central (11a, 11b) les uns à côté des autres à une distance permettant le passage du catalyseur.

6. Réacteur selon la revendication 1 ou l'une des suivantes,
caractérisé en ce que
les tubes gicleurs (9a, 9b) sont entourés par une enveloppe perforée (17a)

7. Réacteur selon la revendication 6,
caractérisé en ce que
l'enveloppe (17a, 17b) est réalisée en métal déployé.

Fig. 1

Fig. 2

Fig. 3

Fig. 4